# FASCICULE DE BREVET EUROPEEN

(11) **EP 3 730 262 B1**
(45) Date de publication et mention de la délivrance du brevet: **06.08.2025**
(21) Numéro de dépôt: 20171404.5
(22) Date de dépôt: 24.04.2020
(51) Int. Cl.: B28B 13/02, G01N 33/38, G01N 3/02, B28B 7/00

(54) **MACHINE D ARASAGE POUR MOULE D ÉPROUVETTE(S) DE MATÉRIAU DE CONSTRUCTION**
NACHSCHNEIDEMASCHINE FÜR EINE FORM ZUR HERSTELLUNG VON BAUMATERIAL-PRÜFSTÜCKEN
TRIMMING MACHINE FOR A MOULD FOR SAMPLE(S) OF CONSTRUCTION MATERIAL

(30) Priorité: 25.04.2019 FR 1904397
(43) Date de publication de la demande: 28.10.2020
(73) Titulaire: Recherches et Realisations Remy, 82000 Montauban (FR)
(72) Inventeur: REMY, Jean-Baptiste, 82130 LAMOTHE CAPDEVILLE (FR); VALES, Benjamin, 82300 CAUSSADE (FR)
(74) Mandataire: Leone, Eloïse

(56) Documents cités:
- CN-A- 107 756 600
- CN-U- 206 690 261
- JP-A- 2007 245 508
- US-A- 1 733 706
- US-A- 4 432 717

## Description

### DOMAINE D'APPLICATION DE L'INVENTION

L'invention a trait au domaine de la cimenterie et notamment au domaine des tests conduits sur des éprouvettes de mortier à des fins de contrôle de qualité.

L'invention concerne plus particulièrement la phase d'arasage et notamment les adaptations permettant de réaliser cette opération de façon optimisée.

### BRÈVE DESCRIPTION DE L'ART ANTÉRIEUR

Un procédé regroupant les différentes phases de ces essais ou tests des matériaux de construction est notamment décrit par la norme NF EN 196-1.

Cette norme décrit ainsi la fabrication d'éprouvettes en mortier c'est-à-dire dans un mélange de ciment de sable et d'eau, puis, la façon dont les éprouvettes doivent être testées. Les éprouvettes décrites adoptent la forme d'un prisme à section rectangulaire.

Dans le cadre de la réalisation de ces éprouvettes, il est prévu que le mortier soit coulé dans un moule. Ce moule est un moule ouvert comprenant classiquement trois empreintes d'éprouvettes. De par la géométrie du moule, pour chaque éprouvette, une seule des six faces n'est pas définie par une surface interne du moule.

Afin de réaliser un « bon » moulage et former cette face de l'éprouvette, le dessus du moule fait l'objet d'une opération d'arasage qui élimine le surplus de mortier et met ainsi à niveau avant séchage, le mortier et le bord haut du moule. Cette opération est sensée garantir l'obtention d'éprouvettes identiques.

La demanderesse a constaté que la répétabilité des résultats obtenus lors des phases de test des éprouvettes pouvait dépendre de cette opération d'arasage. Ainsi, la demanderesse a constaté que cette opération d'arasage, qui est dans le processus de fabrication des éprouvettes, a une incidence sur la résistance à la compression des échantillons.

Or, cette opération d'arasage est classiquement réalisée manuellement. L'absence de l'opérateur ou son remplacement sont donc susceptibles d'influer sur lesdits résultats.

Il existe dans l'art antérieur une pluralité de machines assurant l'automatisation d'opérations destinées à la fabrication de produits en matériau de construction telles celles décrites dans les documents US1733706, CN107756600, JP2007245508 et US4432717 mais ces machines ne constituent pas une solution technique pour l'arasage d'un moule d'éprouvette(s) de matériau de construction notamment dans un cadre normalisé associé aux essais en laboratoire.

Le document CN 206 690 261 U divulgue une machine d'arasage du volume du matériau excédentaire remplissant un moule à éprouvette(s) suivant le préambule de la revendication 1.

### BRÈVE DESCRIPTION DE L'INVENTION

Partant de cet état de fait, la demanderesse a mené des recherches pour améliorer la répétabilité de cette opération d'arasage afin de minimiser la dispersion des résultats obtenus lors des phases de test des éprouvettes.

Ces recherches ont abouti à la conception et à la réalisation d'une machine automatisée d'arasage pour moule d'éprouvette(s) de matériau de construction permettant d'apporter une solution aux inconvénients constatés.

Cette machine est destinée à l'arasage des moules d'éprouvettes de toute forme et de tout matériau. Le moule peut proposer une ou plusieurs empreintes à éprouvettes.

La machine d'arasage de l'invention est telle que définie dans la revendication 1.

En automatisant les mouvements, une telle machine permet de reproduire à l'identique les paramètres de l'arasage et d'obtenir la répétabilité recherchée des caractéristiques des éprouvettes. Elle permet en outre de définir et contrôler les courses, les vitesses, les accélérations dudit mouvement. Ces premiers allers-retours sont perpendiculaires à l'axe longitudinal de la raclette.

Cette mobilité motorisée permet notamment d'incliner différemment la raclette selon le sens de l'arasage. La définition de cette orientation permet de s'adapter au matériau, à la vitesse, etc...Ainsi, l'angle d'inclinaison de la raclette est variable et réglable durant l'arasage d'un même moule selon que la translation soit dans le sens aller ou dans le sens retour.

Les allers-retours selon l'axe longitudinal de la raclette ou cisaillement sont réalisés en même temps que les allers-retours réalisés dans le sens perpendiculaire. De plus, l'amplitude du cisaillement est variable et réglable durant l'arasage d'un même moule.

Selon une autre caractéristique particulièrement avantageuse de l'invention, lors du mouvement relatif en translation du bord inférieur de la raclette sur le bord supérieur du moule, la table portant le moule est mobile et la raclette sur cet axe est fixe. Un tel choix a pour avantage de faciliter le dimensionnement de la machine et permet d'en limiter l'encombrement.

Selon une autre caractéristique particulièrement avantageuse de l'invention, la raclette est guidée en rotation au moyen d'un portique mobile en translation perpendiculairement au bord supérieur du moule. Ce déplacement permet à la raclette de s'éloigner ou de se rapprocher du moule. Il facilite également la rotation de la raclette lors du changement de sens de l'arasage. Enfin, il simplifie l'installation et l'extraction du moule.

Selon un mode de réalisation préféré mais non limitatif, cette translation est motorisée.

Selon une autre caractéristique particulièrement avantageuse de l'invention, ledit portique mobile comprend un moyen de pré-charge, composé de ressorts, pour maintenir le contact entre ladite raclette et le rebord supérieur du moule.

Selon un mode de réalisation préféré, la valeur de la pré-charge imposée par la raclette sur le moule est réglable. Le réglage de la tension desdits ressorts permet de régler la pré-charge. Cette pré-charge peut ainsi être contrôlée dynamiquement et être ajustée.

Selon un mode de réalisation préféré, ledit portique mobile disposé au-dessus de la table assure le support, l'orientation, le guidage et l'entraînement de la raclette.

Selon une autre caractéristique particulièrement avantageuse de l'invention, ladite table supporte un cadre intermédiaire encadrant le moule et supportant au moins une bavette de nettoyage avec laquelle, la raclette vient en contact pour se libérer du matériau y adhérant.

Selon un mode de réalisation préféré mais non limitatif, la machine comporte deux bavettes de nettoyage disposées sur le cadre intermédiaire de part et d'autre du moule pour correspondre avec les deux extrémités de course de sorte que la raclette vienne en contact avec une bavette à la fin de chaque mouvement en translation horizontale.

Selon une autre caractéristique particulièrement avantageuse de l'invention, sous la bavette de nettoyage, le cadre intermédiaire est équipé d'une rigole de récupération du matériau détaché par la bavette. La machine peut ainsi être nettoyée par extraction du cadre intermédiaire et nettoyage de ce dernier.

Le portique mobile comprend un moyen de guidage et d'entraînement de la raclette pour ses mouvements d'aller-retour en translation selon son axe longitudinal.

Selon un mode de réalisation préféré mais non limitatif, cette translation longitudinale de la raclette est motorisée.

Selon un mode de réalisation préféré, l'ensemble des mouvements est motorisé.

Selon l'invention, les différents mouvements sont motorisés au moyen de moteurs pas-à-pas.

Cette technologie de motorisation permet de réaliser des déplacements précis et contrôlés. Le module de commande permet en outre d'enregistrer lesdits réglages et de proposer plusieurs programmations d'arasage.

En motorisant l'ensemble des mouvements nécessaires à l'arasage, la machine de l'invention permet donc d'optimiser la répétabilité d'une pluralité d'arasages différents issus de réglages différents enregistrés dans des programmations différentes mises à disposition de l'utilisateur par le module de commande.

La machine réunissant les différentes caractéristiques ci-dessus décrites est une machine quatre axes (trois translations, une rotation), dont les différents mouvements sont dynamiques (ils peuvent évoluer au cours de l'opération) et asservis (en position, vitesse et accélération), où il est possible d'ajuster dynamiquement la pression appliquée par la règle sur le moule et où la règle est nettoyée.

Les concepts fondamentaux de l'invention venant d'être exposés ci-dessus dans leur forme la plus élémentaire, d'autres détails et caractéristiques ressortiront plus clairement à la lecture de la description qui suit et en regard des dessins annexés, donnant à titre d'exemple non limitatif, un mode de réalisation d'une machine d'arasage conforme à l'invention.

### BRÈVE DESCRIPTION DES DESSINS

La figure 1 est un dessin schématique d'une vue extérieure en perspective d'un mode de réalisation d'une machine d'arasage conforme à l'invention ;
La figure 2 est un dessin schématique d'une vue extérieure de face de la machine de la figure 1 ;
La figure 3 est un dessin schématique d'une vue de côté en coupe de la machine de la figure 1 selon le plan de coupe A-A défini sur le dessin de la figure 2 ;
La figure 4 est un dessin schématique d'une vue de détail de côté en coupe de la machine de la figure 1 avec la raclette en position droite en contact avec le bord supérieur du moule ;
La figure 5 est un dessin schématique d'une vue de détail en perspective du portique mobile soutenant, guidant et entraînant la raclette ;

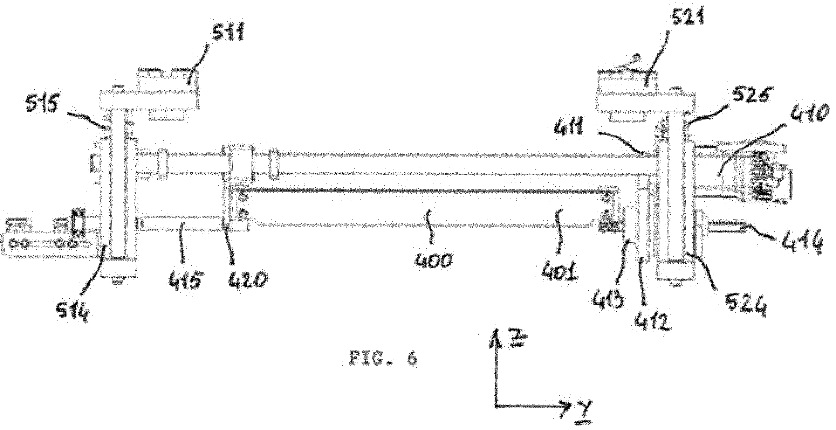

La figure 6 est un dessin schématique d'une vue de détail de face du portique mobile de la figure 5 ;
La figure 7 est un dessin schématique d'une vue de détail de côté de la raclette adoptant une certaine inclinaison ;
La figure 8 est un dessin schématique d'une vue de détail de côté de la raclette adoptant une inclinaison opposée à celle de la figure 7.

### DESCRIPTION D'UN MODE DE RÉALISATION

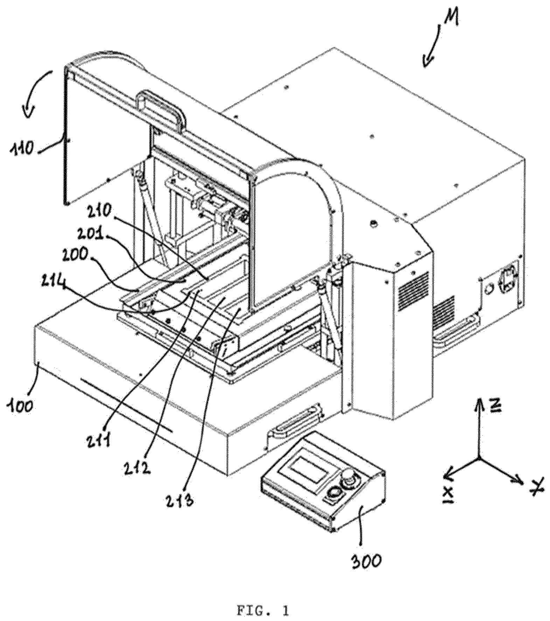

Comme illustrée sur le dessin de la figure 1, la machine d'arasage référencée M dans son ensemble, comprend un bâti 100 accueillant en face avant un poste de travail où une table 200 est mobile horizontalement selon l'axe X.

Cette table 200 permet l'accueil d'un moule 210 composé de trois empreintes à éprouvettes 211, 212, 213. Ces empreintes 211, 212, 213 sont ouvertes et présentent un même rebord supérieur 214. Elles sont pleines lors du fonctionnement de la machine M. Le remplissage du moule 210 est réalisé lors d'une opération préalable non illustrée. Une fois rempli, le moule 210 est positionné sur la table 200. Comme illustrée, la table 200 accueille un cadre intermédiaire 201 encadrant le moule 210.

Un module de commande 300 assure la gestion des programmes d'arasage. Il pilote les différents actionneurs de la machine M en fonction du programme sélectionné par l'utilisateur.

Comme illustrées, l'ouverture ou la fermeture d'un hayon 110 articulé par rapport à la face avant du bâti 100 permet de fermer ou d'ouvrir le poste de travail où se déroule l'arasage et où évolue la table 200 afin :
- de fermer l'accès pendant l'arasage à des fins de protection ;
- de donner accès pendant les opérations connexes (positionnement, enlèvement du moule, maintenance, etc...).

Selon une caractéristique particulièrement avantageuse non illustrée, le poste de travail comporte un moyen d'éclairage interne facilitant les opérations et la vision dans le poste de travail.

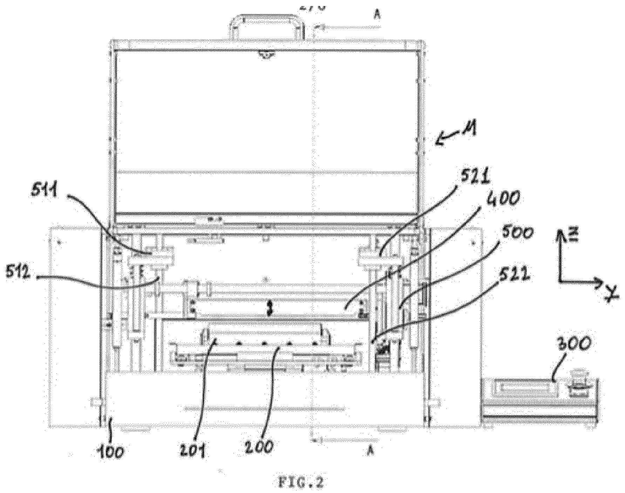

Comme illustrée sur le dessin de la figure 2, une raclette ou règle à araser 400 évolue au-dessus de la table et est associée à un portique mobile 500.

Selon un mode de réalisation préféré mais non limitatif, la surface supérieure du cadre intermédiaire 201 est située à quelques millimètres au-dessous du bord supérieur 214 du moule 210 pour que la raclette 400 soit toujours en contact avec le moule 210. Ce jeu peut être réglé par des vis de réglage.

Le portique mobile 500 guide et entraîne la raclette 400 selon :
- un mouvement de translation verticale selon l'axe Z pour l'éloigner ou la rapprocher de la table 200 et donc du moule 210 ;
- un mouvement de translation horizontale selon l'axe Y pour le cisaillement du matériau ici du mortier ;
- un mouvement de rotation selon l'axe Y pour modifier l'angle d'arasage.

Ces mouvements associés aux mouvements en translation horizontale de la table 200 et donc du moule 210 selon l'axe X permettent de reproduire les mouvements d'une opération d'arasage.

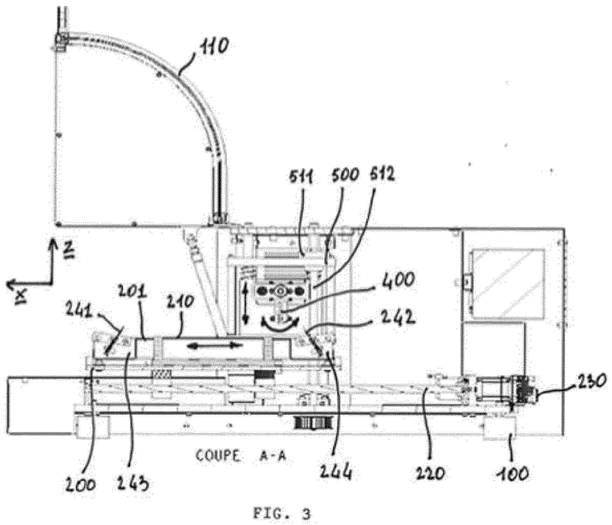

Comme illustrée sur le dessin de la figure 3, ladite table 200 est reliée en partie basse par une liaison hélicoïdale à une tige filetée trapézoïdale 220 entraînée en rotation au moyen d'un moteur pas à pas 230. Cet entraînement assure les mouvements d'aller-retour en translation horizontale selon l'axe X.

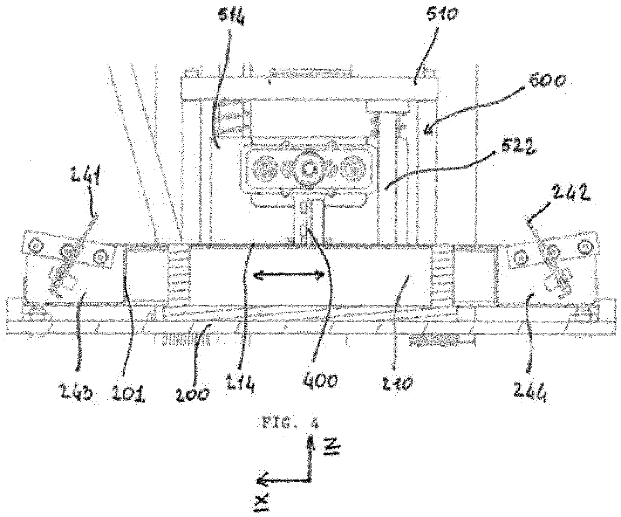

Comme illustrée sur cette figure 3 et sur la figure 4, ledit cadre intermédiaire 201 est équipé en outre de deux bavettes de nettoyage 241 et 242 comprenant une partie souple saillant vers le haut et avec laquelle la raclette 400 est en contact lors des fins de course en translation de la table 200. Pour se faire, ces bavettes 241 et 242 sont disposées sur le cadre intermédiaire 201 au niveau des extrémités de la table 200 de part et d'autre de la position du moule 210.

Les mouvements en translation de la table 200 (et donc du cadre intermédiaire 201 disposé dessus) selon l'axe X sont définis de façon à ce que la raclette 400 dépasse la fin du moule 210 et viennent en contact avec lesdites bavettes 241 et 242. Le matériau en excédent raclé par la raclette 400 et adhérant à cette dernière se détache alors et tombe. Ce matériau est recueilli dans des rigoles 243 et 244 disposées sous les bavettes 241 et 242 et ménagées dans le cadre 201.

Les bavettes 241 et 242 présentent une certaine inclinaison par rapport à la raclette 400. Lorsqu'elle est en contact avec la bavette, l'automatisation des mouvements permet à la raclette 400 de suivre en outre un mouvement vers le haut en fin de course afin de faciliter le détachement du matériau y adhérant après chaque passage.

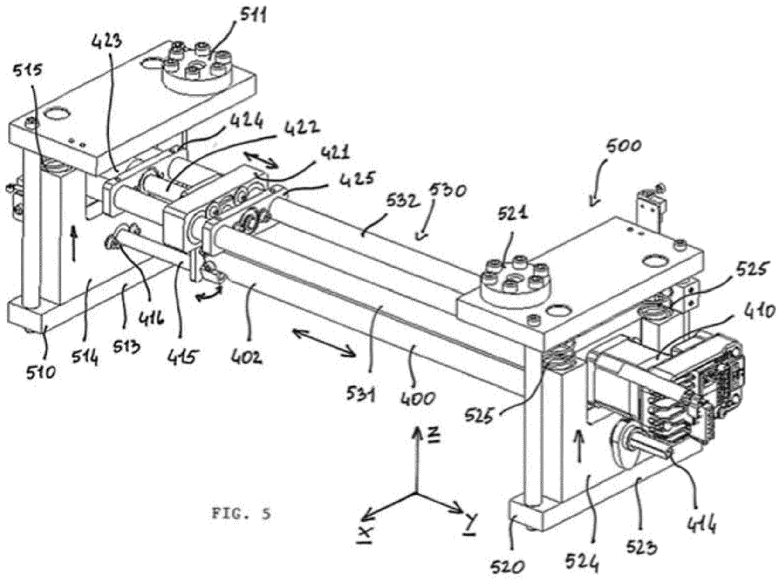

Comme illustrée sur les figures 2, 3 et 5, le portique mobile 500 assurant le support, le guidage et l'entraînement de la raclette 400 au-dessus de la table mobile 200 et donc du moule 210, est mobile en translation verticale selon l'axe Z. Il est disposé au-dessus de la table 200 et est composé de deux équipages mobiles 510 et 520 reliés par une poutre transversale 530.

La mobilité verticale du portique 500 est mise en œuvre par une liaison hélicoïdale avec deux tiges filetées verticales. Selon le mode de réalisation illustré, chaque équipage mobile 510 et 520 est équipé d'un manchon taraudé 511 et 521 coopérant avec une tige filetée trapézoïdale verticale 512 et 522, les deux tiges 512 et 522 étant synchronisées mécaniquement.

Ces équipages mobiles 510 et 520 comportent un cadre 513 et 523 à l'intérieur duquel évolue une partie mobile ou flottante selon un axe vertical 514 et 524 soutenant les sous-ensembles orientant angulairement la raclette 400 et guidant et entraînant en translation selon l'axe Y cette dernière.

Lesdits manchons taraudés 511 et 521 sont associés au cadre 513 et 523 de chaque colonne mobile 510 et 520.

La mobilité des parties mobiles 514 et 524 est accompagnée par des ressorts 515 et 525 qui maintiennent en position basse les parties mobiles. Ainsi, lorsque le mouvement en rotation des tiges filetées 512 et 522 amène la raclette 400 en contact avec le bord supérieur du moule 210 comme illustrée sur le dessin de la figure 4, les parties mobiles 514 et 524 se soulèvent et subissent la contrainte des ressorts qui tendent à les ramener vers le bas.

La raclette 400 est ainsi en pré-charge sur le bord supérieur 514 du moule 210 ce qui garantit le contact et donc l'arasage lors du mouvement de la table 200.

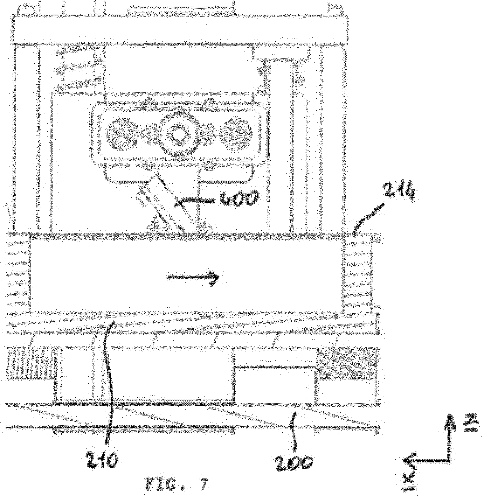

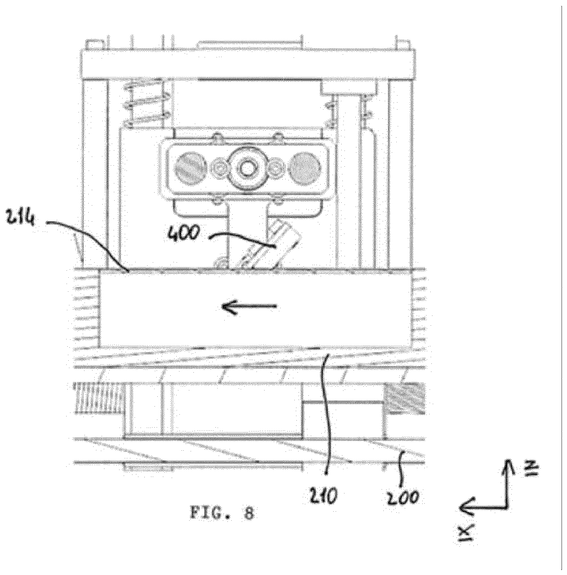

L'orientation angulaire de la raclette 400 est illustrée par la comparaison entre les dessins des figures 4, 7 et 8. Comme illustrée sur les figures 7 et 8, cette orientation change selon le sens d'arasage. Elle est mise en œuvre par un entraînement en rotation de la raclette 400 qui doit non seulement être orientée angulairement mais également doit assurer un mouvement de cisaillement c'est-à-dire d'aller-retour selon l'axe Y pendant la phase d'arasage. Pour ce faire, une liaison pivot glissant est mise en oeuvre entre les parties mobiles 514 et 524 et la raclette 400 pour autoriser le réglage de l'orientation angulaire et le mouvement en translation d'aller-retour.

Un moteur met en mouvement un pignon 411 qui met en mouvement une roue dentée 412 dont le centre forme un manchon de glissement 413 pour une première portion d'arbre horizontal à section prismatique 414 reliée à une extrémité 401 de la raclette 400. L'autre extrémité 402 de la raclette 400 est liée à une deuxième portion d'arbre horizontal 415 qui elle est cylindrique mais coaxiale à la première et glisse dans un palier 416 logé dans la partie mobile 514. La rotation de la roue dentée 412 oriente la raclette sans empêcher son glissement longitudinal.

Comme illustré, l'axe de rotation de la raclette défini par la position des portions d'arbre 414 et 415 est positionné au niveau du rebord bas de la raclette 400. Ce positionnement permet de minimiser le couple nécessaire pour maintenir l'orientation de la raclette 400 durant l'arasage.

L'entraînement en translation pour les mouvements d'aller-retour de la raclette selon son axe longitudinal est mis en œuvre par une fourchette d'entraînement 420 dont l'extrémité basse est liée par une liaison pivot à la portion d'arbre 415 et dont l'extrémité haute est fixé à une pièce mobile 421 ménagée d'une âme creuse taraudée de façon à coopérer avec une tige trapézoïdale horizontale 422 dont la rotation dans un sens puis dans l'autre assure un mouvement de va-et-vient de la pièce mobile 421. Le mouvement de va-et-vient de la pièce mobile 421 est transmis au moyen de la fourchette 420 à la raclette 400 qui peut ainsi réaliser les mouvements de cisaillement prévus pour l'arasage. Un moteur pas-à-pas 423 entraîne en rotation ladite tige trapézoïdale 422.

Le guidage de ce mouvement en translation de la pièce mobile 421 est mis en œuvre par une paire de tiges lisses horizontales 531 et 532 reliant les deux parties mobiles 514 et 524 des équipages mobiles 510 et 520. Le guidage en rotation de la tige trapézoïdale horizontale 422 est mis en œuvre par deux paliers 424 et 425 fixés auxdites tiges entre lesquels évolue la pièce mobile 421.

Un exemple de procédé de fonctionnement de la machine d'arasage est décrit ci-après.

Une fois rempli de mortier, le moule 210 est placé sur la table 200. La raclette 400 est alors en position haute. L'opérateur ferme de hayon 110 et lance à partir du module de commande 300 un des programmes d'arasage proposés. Le module de commande 300 commande alors les différentes mises en mouvement.

La raclette 400 est orientée angulairement par le moteur 410. La mise en mouvements des tiges filetées 512 et 522 déplace vers le bas le portique mobile 500 et met en contact la raclette orientée 400 avec le rebord supérieur 214 du moule 210. Les parties mobiles sont alors légèrement soulevées et contraintes par les ressorts 515 et 525 pour que la raclette 400 reste en contact avec le rebord supérieur 214 du moule 210.

La table 200 est alors mise en mouvement en translation horizontale selon l'axe X par le moteur 230. Simultanément le moteur 423 assure le mouvement horizontal en va-et-vient de la raclette 400.

Une fois la table 200 arrivée en fin de course, la raclette 400 vient au contact d'une bavette pour se libérer du mortier resté collé. La table 400 est arrêtée en translation. Les tiges filetées verticales 512 et 522 sont alors actionnées pour faire monter le portique mobile 500. Ce mouvement finalise le nettoyage de la raclette 400 et autorise le moteur 410 à changer son orientation. Une fois son orientation inversée, les tiges filetées verticales 512 et 522 sont actionnées de façon à remettre la raclette 400 au contact du rebord supérieur 214 ou de la table 200. Cette dernière est alors entraînée en translation dans le sens contraire.

Ce cycle peut être répété jusqu'à atteindre un arasage satisfaisant.

Cette machine motorise et rend automatiques tous les mouvements liés à l'arasage. Elle rend réglable l'ensemble des paramètres de cet arasage (course, vitesse, accélération, inclinaison, etc...) en utilisant des moteurs pas-à-pas.

Conformément à l'invention, elle optimise ainsi la répétabilité des actions menées lors de l'opération d'arasage.

On comprend que la machine qui vient d'être ci-dessus décrite et représentée, l'a été en vue d'une divulgation plutôt que d'une limitation. Bien entendu des aménagements pourront être apportés à la machine sans pour autant sortir du cadre de l'invention tel que défini par les revendications ci-jointes.

## Revendications

1. Machine d'arasage (M) du volume du matériau excédentaire remplissant un moule (210) à éprouvette(s), la machine (M) comprenant
- ledit moule (210) comprenant un bord supérieur (214),
- une raclette (400) dont le bord inférieur vient en contact avec le bord supérieur (214) du moule (210) à des fins d'arasage,
- une table (200) accueillant le moule à éprouvette(s) (210) contenant ledit matériau, et
- un module de commande (300) contrôlant la motorisation du mouvement relatif en translation soit de la table (200) par rapport à la raclette (400) soit de la raclette (400) par rapport à la table (200),
afin de réaliser une pluralité d'allers-retours en translation du bord inférieur de la raclette (400) sur le bord supérieur (214) du moule (210), la raclette (400) étant mobile suivant un mouvement:
- en rotation selon un axe parallèle à son axe longitudinal afin d'orienter sa position angulaire par rapport au rebord (214) du moule (210) et la fixer, et
- en translation selon un axe parallèle à son axe longitudinal afin de lui imprimer, une fois en contact avec le rebord supérieur (214) du moule (210), un mouvement de cisaillement constitué d'allers-retours selon son axe longitudinal, **caractérisée en ce que** les différents mouvements sont motorisés au moyen de moteurs pas-à-pas.

2. Machine (M) selon la revendication 1, **CARACTÉRISÉE PAR LE FAIT QUE** la machine est configurée de sorte que lors du mouvement relatif en translation du bord inférieur de la raclette (400) sur le bord supérieur (214) du moule (210), la table (200) portant le moule (210) est mobile et la raclette (400) est fixe.

3. Machine (M) selon la revendication 1, **CARACTÉRISÉE PAR LE FAIT QUE** la raclette (400) est guidée en rotation au moyen d'un portique (500) mobile en translation perpendiculairement au bord supérieur du moule permettant à la raclette (400) de s'éloigner ou de se rapprocher du moule (210).

4. Machine (M) selon la revendication 3, **CARACTÉRISÉE PAR LE FAIT QUE** ledit portique mobile (500) comprend un moyen de pré charge, composé de ressorts (515, 525), pour maintenir le contact entre ladite raclette (400) et le bord supérieur (514) du moule (210).

5. Machine (M) selon la revendication 3, **CARACTÉRISÉE PAR LE FAIT QUE** ledit portique mobile (500) comprend un moyen de guidage et d'entraînement de la raclette (400) pour ses mouvements d'aller-retour en translation selon son axe longitudinal.

6. Machine (M) selon l'une quelconque des revendications 1 à 4, **CARACTÉRISÉE PAR LE FAIT QUE** ladite table (200) supporte un cadre intermédiaire (201) encadrant le moule et supportant au moins une bavette de nettoyage (241, 242) avec laquelle la raclette (400) vient en contact pour se libérer du matériau y adhérant.

7. Machine (M) selon la revendication 6, **CARACTÉRISÉE PAR LE FAIT QUE** sous la bavette de nettoyage (241, 242), le cadre intermédiaire (201) est équipé d'une rigole de récupération (243, 244) du matériau détaché par la bavette (241, 242).

## Patentansprüche

1. Maschine (M) zum Abstreichen des Volumens an überschüssigem Material, mit welchem eine Form (210) für Prüfkörper gefüllt ist, wobei die Maschine (M) Folgendes umfasst
- die Form (210), welche eine Oberkante (214) umfasst,
- eine Rakel (400), deren Unterkante zum Zwecke des Abstreichens mit der Oberkante (214) der Form (210) in Kontakt kommt,
- einen Tisch (200), der die Form für Prüfkörper (210) aufnimmt, welche das Material enthält, und
- ein Steuermodul (300), das den Motorantrieb der relativen Translationsbewegung entweder des Tisches (200) gegenüber der Rakel (400) oder der Rakel (400) gegenüber dem Tisch (200) steuert, um eine Mehrzahl von vor- und rückwärts gerichteten Translationsläufen der Unterkante der Rakel (400) auf der Oberkante (214) der Form (210) durchzuführen,
wobei die Rakel im Sinne der folgenden Abläufe beweglich ist:
- einer Drehbewegung um eine Achse, die parallel zu ihrer Längsachse ist, um ihre Winkelstellung gegenüber dem Rand (214) der Form (210) auszurichten und festzulegen, und
- einer Translationsbewegung gemäß einer Achse, die parallel zu ihrer Längsachse ist, um sie, sobald sie mit dem oberen Rand (214) der Form (210) in Kontakt ist, in eine Scherbewegung zu versetzen, welche aus vor- und rückwärts gerichteten Läufen gemäß ihrer Längsachse besteht,
**dadurch gekennzeichnet, dass** die verschiedenen Bewegungen mittels schrittweise wirkender Motoren motorgetrieben sind.

2. Maschine (M) nach Anspruch 1, **DADURCH GEKENNZEICHNET, DASS** die Maschine derart ausgelegt ist, dass bei der relativen Translationsbewegung der Unterkante der Rakel (400) auf der Oberkante (214) der Form (210) der Tisch (200), welcher die Form (210) trägt, beweglich ist und die Rakel (400) feststehend ist.

3. Maschine (M) nach Anspruch 1, **DADURCH GEKENNZEICHNET, DASS** die Drehbewegung der Rakel (400) mittels eines Portalkrans (500) geführt wird, der eine Translationsbewegung rechtwinklig zur Oberkante der Form ausführen kann und es Rakel (400) ermöglicht, sich von der Form (210) zu entfernen oder sich dieser anzunähern.

4. Maschine (M) nach Anspruch 3, **DADURCH GEKENNZEICHNET, DASS** der bewegliche Portalkran (500) ein Vorspannmittel umfasst, welches sich aus Federn (515, 525) zusammensetzt, um den Kontakt zwischen der Rakel (400) und der Oberkante (514) der Form (210) aufrechtzuerhalten.

5. Maschine (M) nach Anspruch 3, **DADURCH GEKENNZEICHNET, DASS** der bewegliche Portalkran (500) ein Mittel zum Führen und Mitnehmen der Rakel (400) für deren vor- und rückwärtsgerichtete Translationsbewegungen gemäß ihrer Längsachse umfasst.

6. Maschine (M) nach einem beliebigen der Ansprüche 1 bis 4, **DADURCH GEKENNZEICHNET, DASS** der Tisch (200) einen Zwischenrahmen (201) trägt, welcher die Form einfasst und mit mindestens einem Reinigungsblatt (241, 242) versehen ist, mit welchem die Rakel (400) in Kontakt kommt, um von dem an ihr haftenden Material befreit zu werden.

7. Maschine (M) nach Anspruch 6, **DADURCH GEKENNZEICHNET, DASS** der Zwischenrahmen (201) unter dem Reinigungsblatt (241, 241) mit einer Rinne (243, 244) zur Gewinnung des Materials versehen ist, welches mittels des Blattes (241, 242) gelöst wurde.

## Claims

1. Machine (M) for shaving the volume of excess material filling a test piece mould (210), the machine (M) comprising
- said mould (210) comprising an upper edge (214),
- a squeegee (400) the lower edge of which comes into contact with the upper edge (214) of the mould (210) for shaving purposes,
- a table (200) accommodating the test piece mould (210) containing said material, and
- a control module (300) controlling the motorization of the relative translational movement of either the table (200) with respect to the squeegee (400) or the squeegee (400) with respect to the table (200), in order to carry out a plurality of translational back-and-forth movements of the lower edge of the squeegee (400) over the upper edge (214) of the mould (210),
the squeegee (400) being mobile according to a movement:
- rotating about an axis parallel to its longitudinal axis in order to orient its angular position relative to the rim (214) of the mould (210) and fix it, and
- in translation along an axis parallel to its longitudinal axis in order to impart to it, once in contact with the upper rim (214) of the mould (210), a shearing movement consisting of back-and-forth movements along its longitudinal axis,
**characterised in that** the different movements are motorised by means of stepping motors.

2. Machine (M) according to claim 1, **characterised in that** the machine is configured such that during the relative translational movement of the lower edge of the squeegee (400) over the upper edge (214) of the mould (210), the table (200) carrying the mould (210) is mobile and the squeegee (400) is fixed.

3. Machine (M) according to claim 1, **characterised in that** the squeegee (400) is guided in rotation by means of a gantry (500) movable in translation perpendicularly to the upper edge of the mould allowing the squeegee (400) to move away from or closer to the mould (210).

4. Machine (M) according to claim 3, **characterised in that** said movable gantry (500) comprises a preloading means, composed of springs (515, 525), for maintaining the contact between said squeegee (400) and the upper edge (514) of the mould (210).

5. Machine (M) according to claim 3, **characterised in that** said movable gantry (500) comprises a means for guiding and driving the squeegee (400) for its translational back-and-forth movements along its longitudinal axis.

6. Machine (M) according to any one of claims 1 to 4, **characterised in that** said table (200) supports an intermediate frame (201) framing the mould and supporting at least one cleaning flap (241, 242) with which the squeegee (400) comes into contact to release the material adhering thereto.

7. Machine (M) according to claim 6, **characterised in that** under the cleaning flap (241, 242), the intermediate frame (201) is equipped with a recovery gutter (243, 244) for recovering the material detached by the flap (241, 242).
